# FASCICULE DE BREVET EUROPEEN

(11) **EP 1 295 623 B1**
(45) Date de publication et mention de la délivrance du brevet: **14.01.2004**
(21) Numéro de dépôt: 02292335.3
(22) Date de dépôt: 24.09.2002
(51) Int. Cl.: A61N 1/37, A61N 1/365, A61B 5/08

(54) **Dispositif médical actif comprenant des moyens de diagnostic du profil respiratoire**
Aktive implantierbare medizinische Vorrichtung mit Mitteln zur Diagnostik des respiratorischen Profiles
Active implantable medical device with diagnostic means of the respiratory profil

(30) Priorité: 24.09.2001 FR 0112239
(43) Date de publication de la demande: 26.03.2003
(73) Titulaire: ELA MEDICAL, F-92541 Montrouge (FR)
(72) Inventeur: Bonnet, Jean-Luc, 45160 Olivet (FR); Limousin, Marcel, 75014 Paris (FR)
(74) Mandataire: Dupuis-Latour, Dominique

(56) Documents cités:
- WO-A-01/41868
- WO-A-93/02744
- WO-A-98/33554
- US-A- 4 827 935
- US-A- 5 245 995
- US-A- 5 974 340

## Description

L'invention concerne les "dispositifs médicaux actifs" tels que définis par la directive 90/385/CEE du 20 juin 1990 du Conseil des communautés européennes.

Elle concerne plus particulièrement le diagnostic des troubles de la fréquence et de l'amplitude respiratoires au moyen de tels appareils.

Elle sera plus particulièrement décrite dans le cas de dispositifs implantables tels que des stimulateurs cardiaques, défibrillateurs et/ou cardioverteurs permettant de délivrer au coeur des impulsions électriques de faible énergie pour le traitement des troubles du rythme cardiaque. On soulignera cependant que ce type de dispositif n'est en aucune façon limitatif de l'invention et que les enseignements de cette dernière sont directement applicables à de nombreux types de dispositif médicaux actifs, de diagnostic et/ou de thérapie.

Les patients souffrant d'insuffisance cardiaque présentent fréquemment une ventilation perturbée. L'incidence des apnées du sommeil est anormalement augmentée, et le profil respiratoire est également souvent modifié. Entre autres, ces patients peuvent présenter des profils périodiques de ventilation, avec des phases d'hyperventilation alternant avec soit des phases de respiration normale ou d'hypoventilation (trouble connu sous l'appellation de "respiration périodique" ou PB, *Periodic Breathing*), soit des pauses respiratoires (trouble connu sous l'appellation de "dyspnée de Cheyne-Stokes" ou "respiration de Cheyne-Stokes", ou encore CSR, *Cheyne-Stokes Respiration*). Les différentes phases alternées ont chacune une durée de quelques cycles à quelques dizaines de cycles respiratoires, c'est-à-dire de quelques secondes ou dizaines de secondes, pouvant même dépasser une minute.

Le diagnostic de ce type de respiration est un élément qui peut permettre de mieux suivre les patients et d'adapter leur traitement.

L'étude clinique montre en effet que l'amélioration de la fonction cardiaque peut faire disparaître la CSR ; plus précisément, il a été démontré que la CSR disparaît progressivement avec l'amélioration de la fonction ventriculaire gauche.

La CSR peut, dans cet esprit, être considérée comme un symptôme indicateur de l'efficacité de la thérapie.

Inversement, du fait que la CSR cause des symptômes par elle-même, et donc peut aggraver les performances d'un coeur insuffisant, il est important de pouvoir détecter la présence de la CSR et en évaluer l'importance, car la réduction de la CSR (soit par l'amélioration de la fonction cardiaque, soit par d'autres types de traitements, par exemple médicamenteux) peut prévenir le développement de dysfonctions cardiovasculaires nocturnes, réduire l'insomnie et le nombre de réveils nocturnes, ce qui peut améliorer la fonction diurne et la qualité de vie du patient.

L'un des buts de l'invention est de proposer un dispositif actif capable de détecter la présence d'un trouble. respiratoire, notamment de type CSR, et notamment d'opérer une discrimination entre les différents types de profils (PB ou CSR), de façon automatique et fiable.

Le moyen diagnostique classique est la polysomnographie, examen lourd qui nécessite une hospitalisation d'une nuit. Étant donné son coût, cet examen est rarement proposé, surtout dans les premières phases de la maladie. Au surplus, ses difficultés de mise en oeuvre ne permettent pas un suivi régulier, qui seul pourrait permettre d'évaluer l'évolution dans le temps du symptôme.

On connaît par ailleurs (WO-A-98/33554) un dispositif médical implantable actif capable de suivre la fréquence respiratoire par analyse de l'impédance intracorporelle. Toutefois, le but visé par ce dispositif est de fournir un indicateur de la survenue d'un oedème pulmonaire, l'accélération du rythme respiratoire étant un symptôme classique de cette pathologie. Ce dispositif n'analyse que le rythme respiratoire (fréquence) et, faute d'une analyse fine de la ventilation, ne permet pas de distinguer les types de profils tels que des troubles de type PB ou CSR.

On connaît également, notamment d'après le EP-A-0 493 222 (Ela Medical) des stimulateurs cardiaques comprenant des moyens de mesure de l'activité respiratoire du patient, plus précisément de la ventilation-minute, ce paramètre étant utilisé pour asservir la fréquence d'application des impulsions de stimulation.

On connaît également, d'après le WO-A-01 41868, un dispositif comprenant : des moyens de mesure d'activité respiratoire, aptes à délivrer un signal de ventilation-minute représentatif de la périodicité et de l'amplitude des cycles respiratoires successifs du patient ; des moyens discriminateurs aptes, par analyse du signal, à opérer une discrimination entre différents types de profils respiratoires et à diagnostiquer un profil respiratoire de type Cheyne-Stokes, ces moyens discriminateurs comprenant des moyens aptes à détecter une alternance de cycles respiratoires d'hyperventilation séparés par des périodes de pause respiratoire ou de périodes d'hypoventilation ou de ventilation normale et, dans ce dernier cas, à discriminer entre périodes de pause respiratoire et périodes d'hypoventilation ou de ventilation normale par analyse des variations au second ordre du signal.

Le dispositif de l'invention concerne un perfectionnement à ce dispositif connu, permettant une discrimination plus précise et plus efficace des profils respiratoires.

À cet effet, le dispositif de l'invention est caractérisé en ce que les moyens discriminateurs comprennent, pour l'analyse des variations au second ordre du signal, des moyens aptes à rechercher des pics de ce signal et à évaluer l'intervalle entre pics successifs.

Les moyens discriminateurs peuvent en particulier comprendre des moyens aptes à comparer à une valeur de seuil l'intervalle entre pics successifs et diagnostiquer un profil respiratoire de type Cheyne-Stokes seulement pour les intervalles de durée supérieure à cette valeur de seuil.

En particulier, la recherche n'est opérée que si la valeur d'amplitude du signal est supérieure à une valeur minimale donnée, et/ou la comparaison n'est opérée que pour des valeurs d'intervalles comprises entre une valeur minimale et une valeur maximale données.

On va maintenant décrire un exemple de mise en oeuvre du dispositif de l'invention, en référence aux dessins annexés.
La figure 1 est un chronogramme montrant les variations à court terme du signal d'impédance, dans le cas d'une ventilation nocturne normale.
Les figures 2 et 3 sont des chronogrammes montrant les variations à plus long terme du signal d'impédance, pour des profils respiratoires pathologiques, respectivement de type CSR et PB.
La figure 4 est un chronogramme montrant les variations du paramètre de ventilation-minute obtenu à partir d'un signal d'impédance tel que celui illustré figure 3.
Les figures 5, 6 et 7 sont des chronogrammes montrant, pour diverses évolutions du signal de ventilation-minute, les durées des pauses respiratoires éventuellement détectées ainsi que les marqueurs CSR ou PB générés le cas échéant par le dispositif.

Pour rechercher des pathologies de type PB ou CSR, et opérer une discrimination entre ces deux types de profils, le dispositif de l'invention recherche une périodicité dans le signal de ventilation-minute, lui-même mesuré à partir des paramètres d'amplitude et de périodicité des cycles respiratoires successifs.

L'analyse cycle à cycle est opérée suivant une méthode standard, par exemple décrite dans le EP-A-0 493 222 précité, auquel on pourra se référer pour de plus amples détails sur la manière dont le signal représentatif de l'activité respiratoire est recueilli et analysé, notamment pour en dériver l'information de ventilation-minute (VE).

La mesure de l'activité respiratoire est en elle-même bien connue ; elle est réalisée entre deux électrodes disposées dans la cage thoracique, ou entre une électrode (par exemple une électrode de stimulation, si le dispositif implanté est un stimulateur cardiaque) et un boîtier du dispositif médical implanté. L'impédance est mesurée par injection d'un courant constant de quelques centaines de microampères, à une fréquence de quelques hertz, typiquement 8 Hz. Cette technique est par exemple décrite par Bonnet JL et coll., "Measurement of Minute-Ventilation with Different DDDR Pacemaker Electrode Configurations", *PACE,* Vol. 21, 98, Part 1, et elle est mise en oeuvre dans les appareils *Chorus RM 7034* et suivants d'ELA Médical.

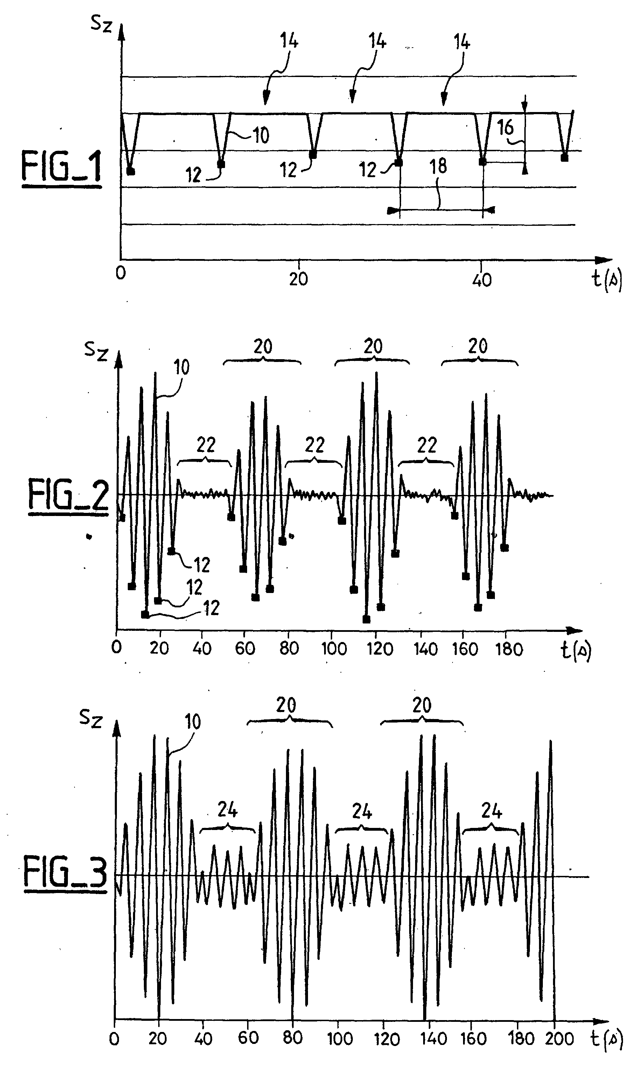

Le dispositif produit ainsi des échantillons successifs d'impédance qui, après filtrage, donnent un signal d'impédance S_{z} tel que celui illustré en 10 figure 1 (une augmentation d'impédance correspondant à une diminution du signal issu du convertisseur analogique/numérique, en raison de la présence d'un inverseur). Ce signal 10 présente des pics d'impédance 12, correspondant aux inspirations (l'impédance augmente par augmentation du volume d'air dans les poumons), séparées par des pauses physiologiques 14, dites pauses expiratoires.

Le dispositif isole les pics d'impédance, c'est-à-dire les creux du signal converti, et détermine pour chaque pic l'amplitude 16 et la période 18 du cycle respiratoire. L'amplitude respiratoire correspond au volume courant du cycle, c'est-à-dire la quantité d'air inspiré ; le rapport entre l'amplitude et la période mesurées pour un même cycle est la mesure de la ventilation-minute VE.

Pour éliminer les artefacts qui produiraient des cycles trop courts et/ou d'amplitude trop faible, seules sont retenues les périodes et les amplitudes situées à l'intérieur d'une plage de valeurs prédéterminée.

La figure 2 montre l'évolution, sur une plus longue durée, du signal d'impédance S_{z} dans le cas d'une respiration de type Cheyne-Stokes (CSR).

Dans ce cas, on observe typiquement des fuseaux d'hyperventilation 20, durant environ 3 à 30 cycles respiratoires (environ 6 à 75 secondes) séparés par des pauses respiratoires longues (apnées) 22, d'une durée pouvant atteindre typiquement 5 à 70 secondes.

La figure 3 est homologue de la figure 2, dans le cas d'une respiration périodique (PB). Le profil est lui aussi périodique, mais les pauses respiratoires 22 sont remplacées par de simples périodes d'hypoventilation ou de ventilation normale 24.

L'étape suivante de l'analyse consiste à évaluer les variations sur le long terme de l'activité respiratoire. On entendra dans la suite par "variation au premier ordre" les variations du paramètre considéré (signal d'impédance) au cours d'un même cycle respiratoire, et par "variation au second ordre" les variations de la ventilation minute VE sur plusieurs cycles ou dizaines de cycles successifs.

L'étape suivante consiste donc à rechercher et analyser une périodicité au second ordre de l'activité ventilatoire, révélatrice d'un profil de type PB ou CSR.

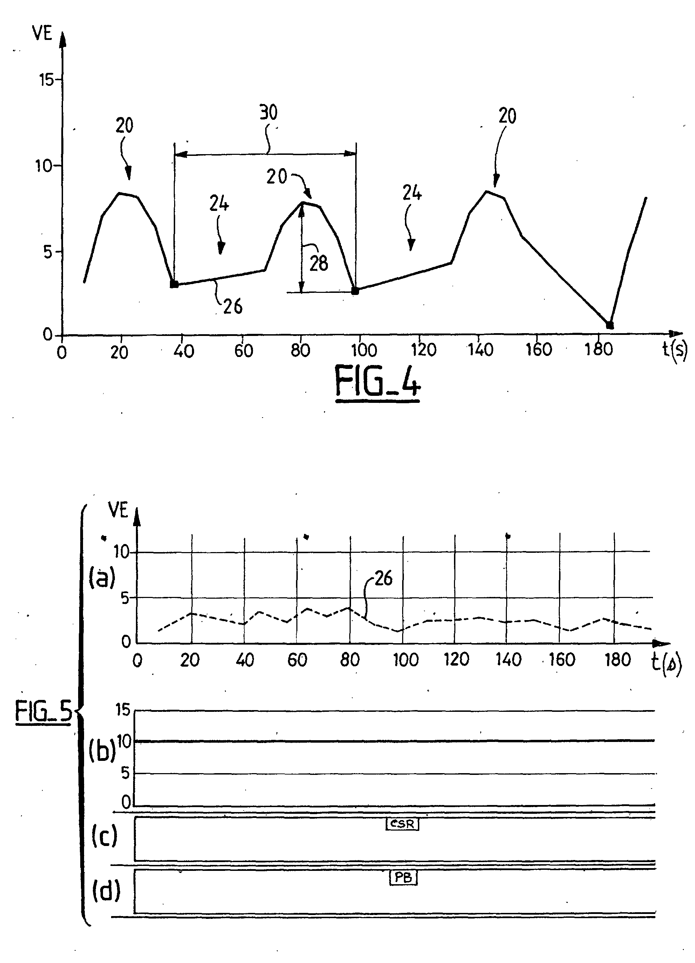

A partir du signal d'impédance indiqué plus haut, on évalue les variations de la ventilation-minute VE au cours du temps, comme illustré par le signal 26 de la figure 4 (et aussi de la ligne (a) des figures 5 à 7).

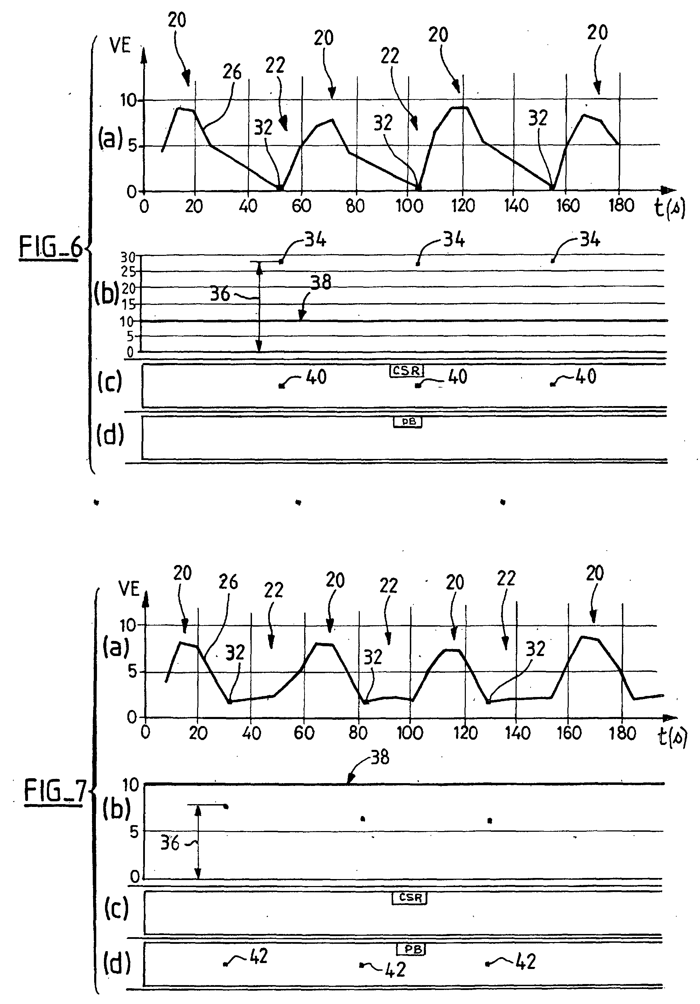

Dans le cas d'une respiration du type PB ou CSR, la ventilation-minute présente, tout comme le signal d'impédance S_{z}, une alternance de périodes d'hyperventilation 20 et de pauses respiratoires 22 (figures 6 et 7) ou de périodes de respiration normale ou d'hypoventilation 24 (figure 4).

Le dispositif évalue l'amplitude 28 et la période 30 des variations au second ordre de la ventilation-minute VE, en appliquant au préalable une sélection de manière à ne retenir que les pics présentant une période minimale (correspondant à la périodicité recherchée des phases successives, typiquement un minimum de 10 secondes par exemple) et une amplitude limite.

Suivant la nature de la respiration, le signal ventilatoire sera plus ou moins cyclique.

Les figures 5 à 7 présentent les trois situations respectives suivantes :
- figure 5 : respiration normale ;
- figure 6 : respiration anormale de type CSR, si présents ;
- figure 7 : respiration anormale de type PB, si présents.

Les quatre chronogrammes de chacune de ces figures illustrent :
- ligne (a) : la variation au cours de temps du signal de ventilation-minute VE (cette ligne correspond au signal illustré par ailleurs figure 4) ;
- ligne (b) : pour chaque pic de ventilation éventuellement trouvé, la représentation quantifiée de la plus longue période respiratoire ;
- ligne (c) les marqueurs de diagnostic de CSR ;
- ligne (d) : les marqueurs de diagnostic de PB.

Dans le cas d'une respiration normale (figure 5), aucun pic de ventilation significatif n'est détecté (compte tenu des limites et d'amplitudes imposées par l'algorithme) et aucun diagnostic n'est établi (absence de marqueur).

Dans le cas d'une respiration de type CSR (figure 6), le signal de ventilation-minute 26 présente une succession de pics de ventilation 32, malgré les critères de limitation prévus par l'algorithme (minimum et maximum sur la durée du cycle au second ordre entre deux pics ventilatoires, et critère d'amplitude minimale sur la variation pour éliminer les cycles trop longs et/ou de trop faible amplitude qui seraient le reflet d'une respiration normale, de faible variation et de longue périodicité, comme dans le cas de la figure 5).

Dans ce cas, pour différencier entre un profil PB et un profil CSR, le dispositif analyse la période la plus longue du cycle ; cette valeur est représentée sur la ligne (b) par un point 34 situé à un niveau 36, situé dans l'exemple illustré entre 25 et 30 secondes. Ce niveau 36 est comparé à un seuil minimal 38, par exemple un seuil de 10 secondes, typiquement : si tel est le cas, comme illustré figure 6, il s'agit bien d'une apnée, et le cycle est donc classé CSR par enregistrement d'un marqueur correspondant 40 (ligne c)) correspondant à un diagnostic positif de CSR.

Inversement, dans le cas d'un rythme de type PB, comme illustré figure 7, la durée 36 de la période respiratoire la plus longue du cycle est inférieure au seuil 38 fixé dans cet exemple à 10 secondes ; le dispositif établit donc un diagnostic positif de PB (marqueur 42) et non plus de CSR.

## Revendications

1. Un dispositif médical actif du type stimulateur cardiaque, défibrillateur, cardioverteur et/ou dispositif multisite, comprenant :
- des moyens de mesure d'activité respiratoire, aptes à délivrer un signal de ventilation-minute (VE) représentatif de la périodicité et de l'amplitude des cycles respiratoires successifs du patient,
- des moyens discriminateurs aptes, par analyse du signal, à opérer une discrimination entre différents types de profils respiratoires et à diagnostiquer un profil respiratoire de type Cheyne-Stokes, ces moyens discriminateurs comprenant des moyens aptes à :
· détecter une alternance de cycles respiratoires d'hyperventilation (20) séparés par des périodes de pause respiratoire (22) ou de périodes d'hypoventilation ou de ventilation normale (24) et
· dans ce dernier cas, à discriminer entre périodes de pause respiratoire et périodes d'hypoventilation ou de ventilation normale par
· analyse des variations au second ordre du signal, c'est-à-dire sur plusieurs cycles ou dizaines de cycles successifs,
dispositif **caractérisé en ce que** les moyens discriminateurs comprennent, pour l'analyse des variations au second ordre du signal, des moyens aptes à rechercher des pics (32) de ce signal (26) et à évaluer l'intervalle (36) entre pics successifs.

2. Le dispositif de la revendication 1, dans lequel les moyens discriminateurs comprennent des moyens aptes à comparer à une valeur de seuil (38) l'intervalle (36) entre pics successifs, et à diagnostiquer un profil respiratoire de type Cheyne-Stokes seulement pour les intervalles de durée supérieure à cette valeur de seuil.

3. Le dispositif de la revendication 1, dans lequel les moyens discriminateurs comprennent des moyens aptes à comparer la valeur d'amplitude du signal à une valeur minimale donnée et à commander conditionnellement les moyens aptes à rechercher des pics lorsque la valeur d'amplitude du signal est supérieure à cette valeur minimale donnée.

4. Le dispositif de la revendication 2, dans lequel la comparaison n'est opérée que pour des valeurs d'intervalles comprises entre une valeur minimale et une valeur maximale données.

## Claims

1. An active medical device of the pacemaker, defibrillator, cardioverter and/or multisite device type, comprising:
- means for measuring respiratory activity, adapted to provide a minute-ventilation signal (VE) representative of the periodicity and amplitude of the successive respiratory cycles of the patient,
- discriminating means adapted, by analyzing the signal, to discriminate between various types of respiratory profiles, and diagnosing a Cheyne-Stokes type respiratory profile, said discriminating means comprising means adapted to:
. detect an alternation of hyperventilation respiratory cycles (20) separated by periods of respiratory pause (22), or periods of hypoventilation or normal ventilation, and
. in this latter case, discriminate between periods of respiratory pause and periods of hypoventilation or normal ventilation by analysis of variations with second order of the signal, namely on several successive cycles or tens of cycles,
said device being **characterized in that** the discriminating means includes, for analyzing the variation of second order of the signal, means adapted to identify peaks (32) of said signal (26) and evaluate the interval (36) between successive peaks.

2. The device of claim 1, wherein the discriminating means comprises means adapted to compare the interval (36) between successive peaks to a threshold value (38), and to diagnose a Cheyne-Stokes type respiratory profile only for the intervals having a duration greater than said threshold value.

3. The device of the claim 1, wherein the discriminating means comprises means adapted to compare the amplitude value of the signal with a given minimal value and conditionally control the means adapted to identify peaks when the amplitude value of the signal is greater than said given minimal value.

4. The device of the claim 2, wherein the comparison is operated only for interval values ranging between a given minimal value and a given maximum value.

## Patentansprüche

1. Aktive medizinische Vorrichtung vom Typus eines Herzschrittmachers, Defibrillators, Cardioverters und/oder einer auf mehrere Orte verteilten Vorrichtung, die aufweist:
- Einrichtung zur Messung der Aktivität der Atmung, dazu geeignet, ein Signal über das Atemminutenvolumen (VE) zu geben, das repräsentativ ist für die Periodizität und die Amplitude der sukzessiven Atemzyklen des Patienten,
- Einrichtungen zur Unterscheidung, dazu geeignet, durch Analyse des Signals zwischen verschiedenen Typen von Profilen der Atmung zu unterscheiden und ein Profil der Atmung vom Typ Cheyne-Stokes zu diagnostizieren, diese Einrichtungen zur Unterscheidung umfassen Einrichtungen, die dazu geeignet sind:
• ein Alternieren von Atemzyklen der Hyperventilation (20), unterbrochen von Perioden der Atempause (22) oder von Perioden der Hypoventilation oder von normaler Atmung (24) zu erkennen, und
• in letzterem Fall zwischen Perioden der Atempause und Perioden der Hypoventilation oder Perioden normaler Atmung zu unterscheiden durch Analyse der Variationen zweiter Ordnung des Signals, das heißt, über mehrere oder mehrere zehn sukzessive Zyklen,
und die Vorrichtung **dadurch gekennzeichnet ist, dass** die Einrichtungen zur Unterscheidung zur Analyse der Variationen zweiter Ordnung des Signals Einrichtungen aufweisen, die dazu geeignet sind, die Spitzen (32) dieses Signals (26) aufzufinden und das Intervall (36) zwischen sukzessiven Spitzen zu evaluieren.

2. Vorrichtung nach Anspruch 1, bei der die Einrichtungen zur Unterscheidung Einrichtungen aufweisen, die dazu geeignet sind, das Intervall (36) zwischen sukzessiven Spitzen mit einem Schwellenwert (38) zu vergleichen, und ein Profil der Atmung vom Typ Cheyne-Stokes allein anhand von Intervallen mit einer Dauer größer als dieser Schwellenwert diagnostiziert.

3. Vorrichtung nach Anspruch 1, bei der die Einrichtungen zur Unterscheidung Einrichtungen aufweisen, die dazu geeignet sind, den Wert der Amplitude des Signals mit einem gegebenen Minimalwert zu vergleichen und abhängig davon die Einrichtungen, die dazu geeignet sind, Spitzen aufzufinden, zu steuern wenn der Wert der Amplitude des Signals größer ist als dieser gegebene Minimalwert.

4. Vorrichtung nach Anspruch 2, wobei der Vergleich nur durchgeführt wird für Intervallwerte, die zwischen einem gegebenen minimalen und einem gegebenen maximalen Wert liegen.
